# EUROPEAN PATENT SPECIFICATION

(11) **EP 2 652 175 B1**
(45) Date of publication and mention of the grant of the patent: **20.09.2017**
(21) Application number: 11848353.6
(22) Date of filing: 14.12.2011
(51) Int. Cl.: C25B 7/00, G01N 27/447, C07H 21/00

(54) **METHOD FOR ELECTROELUTING GENETIC MATERIAL FROM DRIED SAMPLES**
VERFAHREN ZUR ELEKTROELUIERUNG VON GENETISCHEM MATERIAL AUS GETROCKNETEN PROBEN
PROCÉDÉ D'ÉLECTROÉLUTION D'UN MATÉRIAU GÉNÉTIQUE À PARTIR D'ÉCHANTILLONS SÉCHÉS

(30) Priority: 17.12.2010 US 972236
(43) Date of publication of application: 23.10.2013
(73) Proprietor: General Electric Company, Schenectady, NY 12345 (US)
(72) Inventor: FINEHOUT, Erin Jean, Niskayuna New York 12309 (US); NELSON, John Richard, Niskayuna New York 12309 (US); SPOONER, Patrick McCoy, Niskayuna New York 12309 (US)
(74) Representative: Bannan, Sally
(86) International application number: PCT/US2011/064823
(87) International publication number: WO 2012/082849

(56) References cited:
- US-A- 5 187 083
- US-A1- 2010 285 578
- US-B1- 6 503 716
- SIEMBIEDA STEVEN P ET AL: "Improved method to retrieve DNA from dried silver-stained polyacrylamide gels", CLINICAL CHEMISTRY, vol. 44, no. 9, September 1998 (1998-09), pages 1989-1991, XP002722884, ISSN: 0009-9147
- KOSHIBA M ET AL: "The effect of formalin fixation on DNA and the extraction of high-molecular-weight DNA from fixed and embedded tissues", PATHOLOGY RESEARCH AND PRACTICE, vol. 189, no. 1, 1993, pages 66-72, XP009177464, ISSN: 0344-0338
- DUBEAU L ET AL: "SOUTHERN BLOT ANALYSIS OF DNA EXTRACTED FROM FORMALIN-FIXED PATHOLOGY SPECIMENS", CANCER RESEARCH, AMERICAN ASSOCIATION FOR CANCER RESEARCH, US, vol. 46, no. 6, 1 June 1986 (1986-06-01), pages 2964-2969, XP009170519, ISSN: 0008-5472
- GILBERT ET AL.: 'The Isolation of Nucleic Acids from Fixed, Paraffin-Embedded Tissues- Which Methods Are Useful When?' PLOS ONE 20 June 2007, pages 1 - 12, XP002645965

## Description

### CROSS-REFERENCE TO RELATED APPLICATIONS

This application claims priority to United States patent application number 12/972,236 filed 17 December 2010.

### BACKGROUND OF THE INVENTION

The subject matter disclosed herein relates to a solid medium for use in the storage of genetic material from biological samples and more particularly to methods to electroelute genetic material from the biological samples directly from the solid medium.

Various facilities (e.g., research or medical institutions) include storage systems for large collections of genetic material (e.g., DNA) collected from a wide range of sources (e.g., human blood, cell lines, etc.). The genetic material is stored in a safe, convenient, and minimally labor intensive manner within these storage systems for later analysis. For example, solid media, such as Whatman^{®} FTA^{®} substrates, are used to store genetic material from various biological samples, such as tissues or cells. However, extracting genetic material from the solid media involves additional labor, sometimes intensive labor when dealing with numerous samples. For example, extraction of genetic material may include either using detergents or incubating the solid media at high temperatures. The detergent may interfere with analysis of the genetic material, thus additional labor is required to remove the detergent. Also, the higher temperatures may fragment the genetic material. Thus, there is a need to reduce the work required to extract genetic material, while also minimizing the fragmentation of the genetic material.

### BRIEF DESCRIPTION OF THE INVENTION

In a first embodiment, a method for extracting genetic material from a biological sample stored on a solid medium is provided. The method includes obtaining the solid medium, wherein the biological sample is applied on the solid medium, and the solid medium includes chemicals that lyzed the biological sample and preserved the genetic material. The method also includes electroeluting the genetic material directly from the solid medium to a subsequent medium.

In a second embodiment, a method for extracting genetic material from a fixed tissue sample stored on a solid medium is provided. The method includes applying at least a portion of the fixed tissue sample to the solid medium, wherein the solid medium includes chemicals that can lyse the cells or preserve the genetic material. The method further includes electroeluting the genetic material directly from the solid medium to a subsequent medium.

In a third embodiment, a method for extracting DNA from a biological sample stored on a cellulose-based paper is provided. The method includes obtaining the cellulose-based paper, wherein the biological sample is applied on the cellulose-based paper, and the cellulose based paper includes chemicals that lyse the biological sample and preserve the DNA. The method also includes electroeluting DNA directly from the cellulose-based paper into an electrophoresis gel.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
FIG. 1 is a flow chart illustrating a method for extracting genetic material from a biological sample stored on a solid medium in accordance with aspects of the present disclosure;
FIG. 2 depicts a SYBR^{®} Gold stained electrophoresis gel of DNA electroeluted from solid media using pulsed-field gel electrophoresis in accordance with aspects of the present disclosure;
FIG. 3 depicts a SYBR^{®} Gold stained electrophoresis gel of DNA electroeluted from solid media using alkaline gel electrophoresis in accordance with aspects of the present disclosure;
FIG. 4 is a flow chart illustrating a method for extracting genetic material from a fixed tissue sample stored on a solid medium in accordance with aspects of the present disclosure;
FIG. 5 depicts a SYBR^{®} Gold stained electrophoresis gel, using a vertical polyacrylamide gel electrophoresis (PAGE) system, of multiplex PCR amplification of DNA that has been extracted from a human prostate sample fixed in formalin and applied to Whatman^{®} FTA^{®} paper;
FIG. 6 depicts a SYBR^{®} Gold stained electrophoresis gel of DNA electroeluted from solid media using native gel electrophoresis in accordance with aspects of the present disclosure;
FIG. 7 depicts a SYBR^{®} Gold stained electrophoresis gel of DNA electroeluted from solid media using native gel electrophoresis in accordance with aspects of the present disclosure; and
FIG. 8 depicts a SYBR^{®} Gold stained electrophoresis gel of DNA electroeluted from solid media using native gel electrophoresis in accordance with aspects of the present disclosure.

### DETAILED DESCRIPTION OF THE INVENTION

As discussed in detail below, embodiments of the invention include a method for extracting genetic material (e.g., DNA) directly from biological samples stored on a solid medium (e.g., chemically treated cellulose substrate) using electroelution. In one embodiment, the method includes obtaining the solid medium that includes a stored biological sample previously applied and dried on the solid medium. The solid medium includes chemicals that lyse the biological sample and preserve the genetic material. The method also includes electroeluting the genetic material directly from the solid medium to a subsequent medium after removing the chemicals from the solid medium. The subsequent medium may include an electrophoresis gel, a solution, or a capture surface (e.g., a blotting membrane). Alternatively, fixed samples of cells or tissues may be applied to the solid medium. The fixed samples may be processed prior to or after application to the solid medium. For example, processing of the fixed samples may include rehydrating and/or lyzing the fixed sample. Whether fixed or not, the samples may be further processed subsequent to application to the solid medium. In particular, repair of nicks and abasic sites within the genetic material may occur, while the genetic material is in a fixed position on the solid medium. The methods above provide a single platform for lyzing a biological sample, extracting DNA from the biological sample, binding the DNA to a surface, washing the DNA, and eluting high molecular weight (e.g., at least 10 kilobases) as wells as less fragmented DNA. The ability to directly electroelute the genetic material from the solid medium avoids the use of detergents normally used to extract DNA as well as the extra steps necessary to remove the detergent and make the DNA usable for subsequent analysis. In addition, directly electroeluting the genetic material from the solid medium without using high temperatures reduces the fragmentation of DNA. Prior to this invention, researchers have had to insert the solid medium on which the DNA is located directly into subsequent genetic analysis reactions (e.g. PCR) to analyze the DNA that is bound on the solid medium. It can be appreciated that elution of DNA from the solid medium allows for the evaluation of the genetic material in more than just one reaction. Overall, the disclosed embodiments reduce the work of the user in extracting the DNA from the solid medium while improving the quality and availability of the DNA.

Biological samples used in the embodiments below may include physiological/pathological body liquids (e.g., secretions, excretions, exudates, and transudates) or cell suspensions (e.g., blood, lymph, synovial fluid, semen, saliva containing buccal cells, skin scrapings, hair root cells, etc.), liquid extracts or homogenates of cell suspensions of humans and animals; physiological/pathological liquids or cell suspensions of plants; liquid products, extracts or suspensions of bacteria, fungi, plasmids, viruses, etc.; liquid products, extracts, or suspensions of parasites including helminths, protozoas, spirochetes, etc.; human or animal body tissues (e.g., bone, liver kidney, etc.); media from DNA or RNA synthesis; mixtures of chemically or biochemically synthesized DNA or RNA; and any other source in which DNA and/or RNA is or can be in a liquid medium.

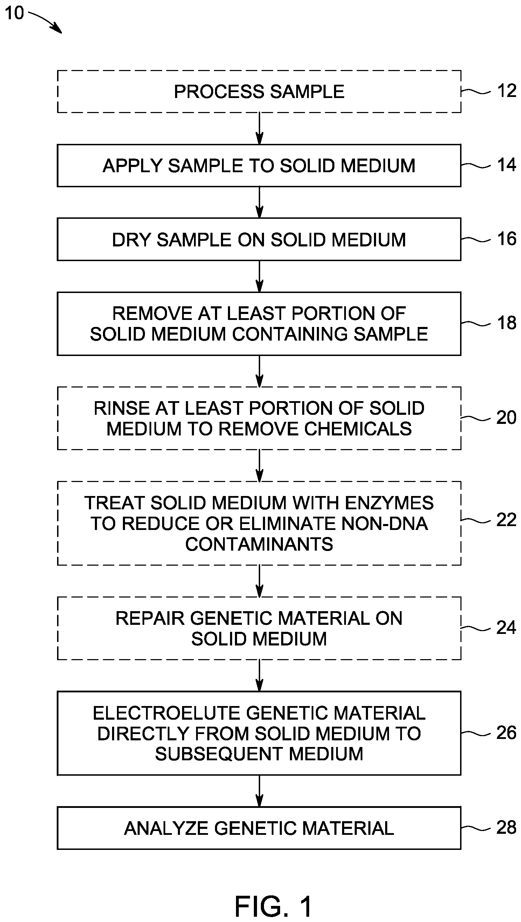

Turning now to the figures, FIG. 1 is a flow chart illustrating an embodiment of a method 10 for extracting genetic material (e.g., DNA) from a biological sample (e.g., tissues, cells, viruses, bacteriophages, or any other sample containing nucleic acid) stored on a solid medium. Processing of the biological sample (block 12) may occur prior to application of the biological sample to the solid medium. For example, in certain embodiments as described in greater detail below, biological samples including cells or tissues may be fixed (e.g., in formalin). Processing of the fixed tissue or cells may include rehydrating the fixed cells or tissues, lyzing the cells or tissues (e.g., with protease), and/or reversing cross-linking between the genetic material (e.g., DNA) and proteins. In certain embodiments, some of the processing, such as reversing cross-linking, may occur subsequent to application of the fixed cells or tissues to the solid medium.

Application of the desired biological sample to the solid medium then occurs (block 14). In one embodiment, the solid medium includes a chemically treated absorbent cellulose-based material. For example, the solid medium may include Whatman^{®} FTA^{®} or FTA^{®} Elute paper (GE Healthcare). The solid medium includes chemicals that lyze the biological sample (e.g., tissues or cells) and/or preserve the genetic material on the solid medium. Indeed, the solid medium and composition of chemicals may be as described in greater detail in U.S. Patent No. 5,976,572, entitled "Dry Solid Medium for Storage and Analysis of Genetic Material," and U.S. Patent No. 5,985,327, entitled "Solid Medium and Method for DNA Storage,". For example, the solid medium may include a weak base (e.g., tris-hydroxymethyl methane (tris)), chelating agent (e.g., ethylene diamine tetracetic acid (EDTA)), anionic surfactant or detergent (e.g., sodium dodecyl sulphate (SDS)), and/or uric acid or urate salt. Upon application to the solid medium, the chemicals lyse the biological sample and denature proteins. In addition, the chemicals inactivate nucleases and pathogens to allow for the preservation and long term storage of the genetic material. Any liquid within the applied sample evaporates after application. Drying of the sample on the solid medium (block 16) occurs after application. For example, the solid medium containing the sample may dry overnight in a desiccator. In certain embodiments, the solid medium containing the sample may be encased in a protective material (e.g., a plastic case) to further preserve the genetic material.

Upon obtaining the solid medium containing the desired dry sample, a portion of the solid medium containing the sample is removed for electroeluting the genetic material (e.g., DNA) from this portion (block 18). In certain embodiments, the entire sample may be used. Prior to electroelution, the portion of the solid medium containing the biological sample may be rinsed to remove the chemicals (block 20). In certain embodiments, rinsing the portion of the sample-containing solid medium includes soaking the solid medium portion in a buffer solution or flowing buffer through or across the solid medium portion. In certain embodiments, the buffer solution includes an alkaline buffer solution (e.g., pH 8.0) including at least tris and EDTA (e.g., T.E.). For example, the solid medium portion with the sample may be soaked one or more times for a fixed time (e.g., 5 minutes) in the alkaline buffer solution. In some embodiments, the buffer solution includes Whatman^{®} FTA^{®} purification reagent from GE Healthcare. For example, the solid medium portion with the sample may be soaked one or more times for a fixed time (e.g., 5 minutes) in the FTA^{®} purification reagent. In other embodiments, both the alkaline buffer solution and the FTA^{®} purification reagent may be used to rinse the sample-containing solid medium portion. For example, the sample-containing solid medium portion may be soaked for a fixed time (e.g., 5 minutes each soaking) twice in the alkaline buffer solution and for a fixed time (e.g., 5 minutes each soaking) twice in the FTA^{®} purification reagent. In alternative embodiments, rinsing the portion of the sample-containing solid medium includes soaking the solid medium portion in water or flowing water through or across the solid medium portion. For example, the solid medium portion with the sample may be soaked one or more times for a fixed time (e.g., 5 minutes) in the water. In certain implementations of electroelution (e.g., electroelution in dialysis tubing), rinsing of the sample-containing solid medium may not be necessary.

Also, prior to electroeluting genetic material from the sample-containing solid medium portion, the solid medium portion with the sample may be treated with enzymes (block 22) to reduce or eliminate non-DNA contaminants (e.g., proteins, lipids, carbohydrates, etc.). For example, an enzymatic solution may include hydrolytic enzymes such as proteases, lipases, and/or glycoside hydrolases.

Further, prior to electroeluting genetic material from the sample-containing solid medium portion, the genetic material (e.g., DNA) may be repaired while fixed in position on the solid medium (block 24). Genetic material, such as DNA, stored on the solid medium may include nicks (i.e., absence of phosphodiester bond between adjacent nucleotides) or abasic sites (also called AP sites, i.e., absence of a purine or pyrimidine base in a nucleotide while retaining the integrity of the phosphodiester ribose backbone) introduced via a fixing agent (e.g., formalin) in pre-processed samples or via other means. In certain embodiments, repairing the genetic material includes applying a solution containing at least DNA polymerase (e.g., *E. coli* DNA polymerase I) and DNA ligase (e.g., T4 DNA ligase or a bacterial DNA ligase) to repair the nicked damage. For example, the DNA polymerase includes DNA polymerase activity in addition to both 3' to 5' exonuclease activity to mediate proofreading and 5' to 3' exonuclease activity to mediate nick translation during DNA repair. DNA polymerase requires dNTP's. The DNA ligase includes enzymatic activity to form a phosphodiester bond between adjacent nucleotides. DNA ligase requires either rATP or NAD. The sample-containing solid medium portion may be incubated with the DNA repair solution at 37°C for 30 minutes, and then incubated at 65°C for 20 minutes. In other embodiments, the DNA repair solution may include other DNA repair enzymes with similar or different DNA repair mechanisms. For example, AP endonuclease (e.g., *E. coli* endonuclease IV) can also be used to remove abasic sites. The abasic site can be cleaved by an AP endonuclease, leaving 3' hydroxyl and 5' deoxyribosephosphate termini. This structure can be subsequently repaired by the combined action of DNA polymerase and DNA ligase.

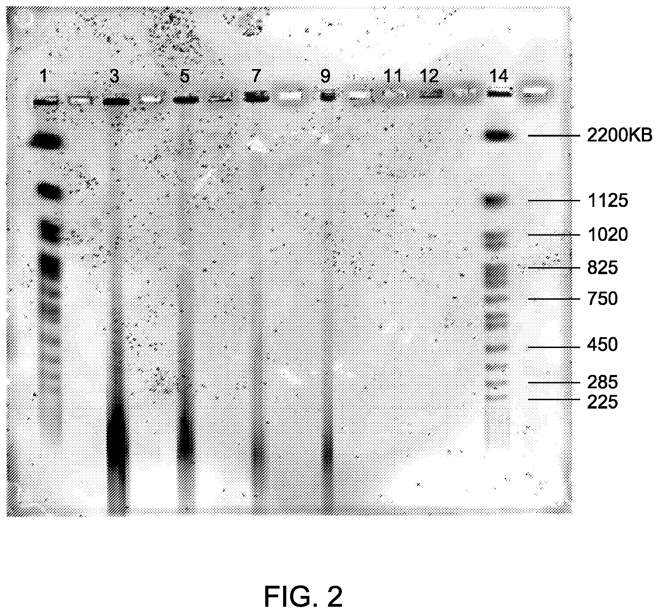

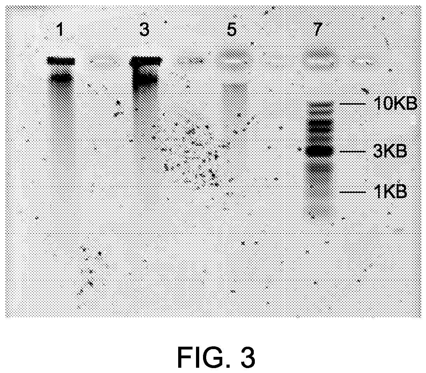

After obtaining the sample-containing solid medium, the genetic material is directly electroeluted from the solid medium to a subsequent medium (block 26). In certain embodiments, the sample-containing solid medium portion may be disposed directly into a well of an electrophoresis gel (e.g., agarose gel), the well sealed with agarose, and the genetic material directly electroeluted into the electrophoresis gel (FIGS. 2 and 3). In other embodiments, the genetic material may be electroeluted into a solution. Electroelution into a solution may occur in a variety of ways. For example, D-tube™ dialyzers (Merck Biosciences Ltd.), dialysis cassettes or tubing, Whatman^{®} Elutrap^{®} Electroelution Systems (GE Healthcare), or other devices may be used to electroelute the genetic material directly from the solid medium into solution. In further embodiments, the genetic material may be directly electroeluted from the solid medium onto a capture surface. The capture surface may be on a blotting membrane (e.g., diethylaminoethyl cellulose (DEAE)).

Following electroelution, the extracted genetic material may be analyzed (block 28). The genetic material extracted from the sample-containing solid medium includes high molecular weight DNA optimally of at least 10 kilobases with few nicks present. Indeed, the DNA extracted in accordance with the present approaches may approach 40 kilobases. In particular, DNA extraction, according to the above embodiment, occurs in the absence of detergents. If detergent were used in extracting the DNA from the solid medium, additional steps would typically be performed to remove the detergent before any subsequent analysis of the extracted DNA could occur. In addition, the extraction of DNA at high temperatures (e.g., 95°C), which fragments DNA, is avoided in the above embodiment. Thus, high molecular weight DNA with minimal fragmentation is made available for analysis with minimal work. The extracted DNA may be used in a variety of analyses including polymerase chain reaction (PCR), single-nucleotide polymorphism analysis, real-time PCR, and other downstream uses of the DNA.

FIGS. 2 and 3 illustrate examples of the electroelution of DNA directly from the sample-containing solid medium into electrophoresis gels. FIG. 2 depicts a SYBR^{®} Gold (Invitrogen) stained electrophoresis gel of DNA electroeluted from solid media using pulsed-field gel electrophoresis. Electrophoresis was in a 1% agarose gel, 0.5X TE, ph 8.0 for 18 hours at 6 V/cm with a switch angle of 120° and switch time from 50-90 seconds. 20 µL of Jurkat cells (suspended at 2 x 10⁶ cells/mL) were applied to either Whatman^{®} FTA^{®} or FTA^{®} Elute paper and dried as described above. Portions of the dried samples of Jurkat cells were removed from the FTA^{®} or FTA^{®} Elute paper and washed according to a variety of methods indicated below. In addition, some of the samples were eluted, prior to loading, into 95°C water. Wet paper portions including the samples of Jurkat cells were placed directly into the wells of the electrophoresis gel and sealed with agarose. The samples electroeleuted into the electrophoresis gel, shown in FIG. 2, are as follows:

Lane 1: *Saccharomyces cerevisiae* chromosomal DNA size marker (BioRad); Lane 3: FTA^{®} paper soaked for 5 minutes in TE, ph 8.0; Lane 5: FTA^{®} paper soaked for 5 minutes in FTA^{®} purification reagent (two times) and soaked for 5 minutes in TE buffer, ph 8.0 (two times); Lane 7: FTA^{®} Elute paper soaked in water for 5 minutes; Lane 9: FTA^{®} Elute paper soaked in water for 5 minutes, then vortexed for 15 seconds; Lane 11: FTA^{®} Elute paper soaked in water for 5 minutes, then eluted into 95°C water for 25 minutes; Lane 12: FTA^{®} Elute paper soaked in water for 5 minutes, then eluted into 95°C water for 25 minutes, and vortexed for 60 seconds; and Lane 14: *Saccharomyces cerevisiae* chromosomal DNA size marker (BioRad). After pulsed-field gel electrophoresis, the DNA was stained with SYBR^{®} Gold and the gel was imaged on a Typhoon™ Imager fluorescent scanner.

The results in FIG. 2 demonstrate the electroelution of high molecular weight DNA from the samples of Jurkat cells directly from the solid medium (i.e., FTA^{®} or FTA^{®} Elute paper) into the electrophoresis gel. The electroeluted DNA is less than 225 kilobases but estimated to be approximately 40 kilobases. FIG. 2 also demonstrates the absence of high molecular weight DNA when attempting to elute the DNA under high temperature conditions (e.g., 95°C). Thus, in order to electroelute high molecular weight DNA from the solid medium directly into the electrophoresis gel only a simple rinse in a buffer solution (e.g., TE buffer or FTA^{®} purification reagent) or water is needed.

FIG. 3 illustrates similar results using alkaline gel electrophoresis. FIG. 3 depicts a SYBR^{®} Gold stained electrophoresis gel of DNA electroeluted from solid media using alkaline standard gel electrophoresis. Electrophoresis was in a 1% agarose gel, 30mM NaOH, 1 mM EDTA, for 2 hours at 150mA. During electrophoresis the gel was covered in a glass plate to prevent diffusion out of the gel. As above, 20 µL of Jurkat cells (suspended at 2 x 10⁶ cells/mL) were applied to Whatman^{®} FTA^{®} paper and dried as described above. Portions of the dried samples of Jurkat cells were removed from the FTA^{®} paper and washed according to a variety of methods indicated below. Wet paper portions including the samples of Jurkat cells were placed directly into the wells of the electrophoresis gel and sealed with agarose. The samples electroeleuted into the electrophoresis gel, shown in FIG. 3, with lanes 1-8 are as follows:

Lane 1: FTA^{®} paper soaked for 5 minutes in TE, ph 8.0; Lane 3: FTA^{®} paper soaked for 5 minutes in FTA^{®} purification reagent (two times) and soaked for 5 minutes in TE buffer, ph 8.0 (two times); Lane 5: purified human DNA (Male, Applied Biosystems); and Lane 7: 1 kb ladder (New England Biolabs). After electrophoresis, the gel was neutralized in a solution containing 1M Tris-HCL (pH=7.7) and 1.5M NaCl for 30 minutes. The DNA was stained with SYBR^{®} Gold and the gel was imaged on a Typhoon™ Imager fluorescent scanner.

The results in FIG. 3 also demonstrate the electroelution of high molecular weight DNA from the samples of Jurkat cells directly from the solid medium (i.e., FTA^{®} or FTA^{®} Elute paper) into the electrophoresis gel. The electroeluted DNA is greater than 10 kilobases. Thus, as above, in order to electroelute high molecular weight DNA from the solid medium directly into the electrophoresis gel only a simple rinse in a buffer solution (e.g., TE buffer or FTA^{®} purification reagent) or water is needed. The fact that the large DNA is also present on an alkaline gel indicates that the electroeluted DNA contains few nicks. As the DNA in an alkaline gel is single stranded, any nicks in the backbone would result in DNA fragments.

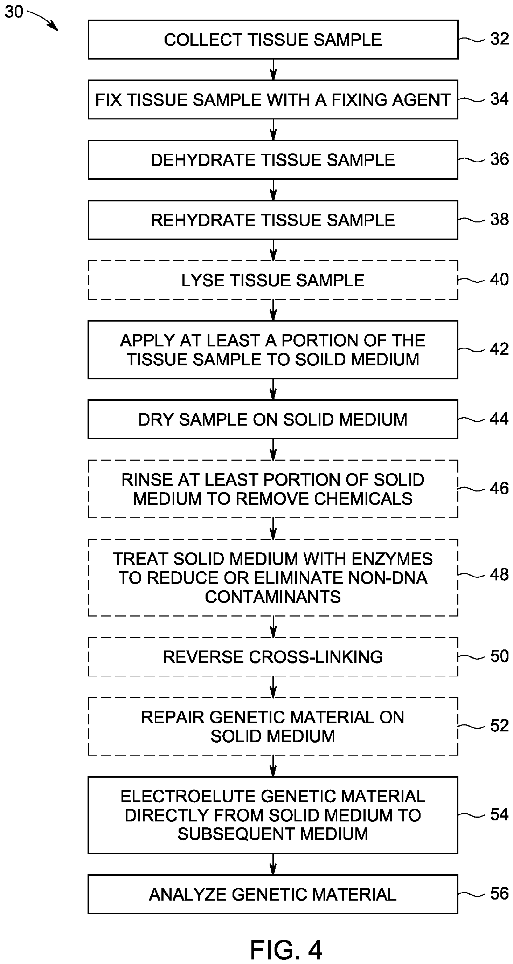

As discussed above, the electroelution of DNA directly from the solid medium also applies to fixed cells or tissues. FIG. 4 is a flow chart illustrating a method 30 for extracting genetic material (e.g., DNA) from a fixed tissue sample stored on a solid medium. Although the method 30 is discussed in terms of fixed tissues samples, the fixed samples may also include fixed cells (e.g., derived from tissues or cell culture lines). To begin, tissue is collected from a source (e.g., a mouse or human) (block 32). Following collection of the tissue, the tissue sample is fixed with a fixing agent (block 34) such as formalin, paraformaldehyde, or other fixing agent, according to methods know to the art. During fixation, the genetic material (e.g., DNA) forms cross-links with proteins. In certain embodiments, such as the embedding of the fixed tissue sample in paraffin, the fixed tissue samples may be dehydrated (block 36) in a series of ethanol washes with the ethanol sequentially increasing in concentration. The dehydrated samples may then be washed in xylene and embedded in paraffin.

After obtaining the desired fixed tissue sample, if the sample was previously dehydrated, then the sample is rehydrated (block 38) in a series of ethanol washes sequentially decreasing in concentration. For example, the fixed tissue sample is rehydrated sequentially for 5 minutes each in 100% ethanol, 75% ethanol, 50% ethanol, 25% ethanol, and distilled water (or T.E.). Following rehydration of the fixed tissue sample, the rehydrated sample may be incubated overnight in 1 M potassium thiocyanate. The fixed tissue sample, rehydrated or not, may be lyzed (block 40), e.g., with a protease, prior to application to the solid medium. For example the fixed tissue sample may be lyzed in a digest buffer solution including Proteinase K (0.5 mg/mL), 50 mM Tris at pH 7.4, 10 mM EDTA, 0.5% SDS, and 50 mM NaCl at 55°C for at least three hours. In certain embodiments, the fixed tissue sample may be lysed via chemicals present on the solid medium as described above.

Application of at least a portion of the desired fixed tissue sample to the solid medium then occurs (block 42). The sample of fixed is applied to the solid medium via such methods as rubbing the sample against the media, pressing the sample against the media, etc. The solid medium is as described above. The solid medium includes chemicals to preserve the genetic material on the solid medium. In certain embodiments, where lyzing of the fixed tissue is desired subsequent to application of the sample, the solid medium includes chemicals that lyze the fixed tissue sample as described above. Any liquid within the applied tissue sample evaporates after application. Drying of the sample on the solid medium (block 44) occurs after application. For example, the solid medium containing the sample may dry overnight in a desiccator. In certain embodiments, the solid medium containing the sample may be encased in a protective material (e.g., a plastic film) to further preserve the genetic material.

Upon obtaining the solid medium containing the desired dry, fixed tissue sample, a portion of the solid medium containing the sample is removed for electroeluting the genetic material (e.g., DNA) from this portion. In certain embodiments, the entire sample may be used. Prior to electroelution, the portion of the solid medium containing the sample may be rinsed to remove the chemicals (block 46). In certain embodiments, rinsing the portion of the sample-containing solid medium includes soaking the solid medium portion in a buffer solution. As described above, the buffer solution may include an alkaline buffer solution (e.g., TE) or FTA^{®} purification reagent. For example, the sample-containing solid medium portion may be soaked for a fixed time (e.g., 5 minutes each soaking) twice in the FTA^{®} purification reagent and for a fixed time (e.g., 5 minutes each soaking) twice in the alkaline buffer solution. However, in certain embodiments, the rinses may occur only in the alkaline buffer solution, only in the FTA^{®} purification reagent, or only in water as described above. As previously mentioned, in certain embodiments of electroelution (e.g., electroelution in dialysis tubing), rinsing of the sample-containing solid medium may not be necessary.

As mentioned above, prior to electroeluting genetic material from the sample-containing solid medium portion, the solid medium portion with the fixed sample may be treated with enzymes (block 48) to reduce or eliminate non-DNA contaminants (e.g., proteins, lipids, carbohydrates, etc.). For example, an enzymatic solution may include hydrolytic enzymes such as proteases, lipases, and/or glycoside hydrolases.

In addition, prior to electroeluting, the cross-linking between the genetic material and protein may be reversed (block 50) subsequent to applying the fixed tissue sample to the solid medium. For example, a crosslink repair buffer including a primary amine (e.g., bicine, pH 8.5) or other nucleophile with a pH higher than 7.0 (preferably with a pH between 7.5-10). To reverse the cross-links, the sample-containing solid medium portion is incubated in the crosslink repair buffer (e.g. at 65°C for 20 hours). The primary amine or nucleophile reacts with the portion of the crosslink that comes from the aldehyde that originally formed the cross-links (between the genetic material and/or the protein) and reverses these cross-links. Alternatively, a sulfhydryl reagent can be used in place of amine, which will also react with the portion of the crosslink that is derived from the aldehyde. This reaction occurs more rapidly than amine-based nucleophile when performed at a pH below 7. In certain embodiments, as mentioned above, the reversal of the cross-linking may occur prior to application of the fixed tissue sample to the solid medium. Further, prior to electroeluting genetic material from the sample-containing solid medium portion, the genetic material (e.g., DNA) may be repaired while fixed in position on the solid medium (block 52) as described above.

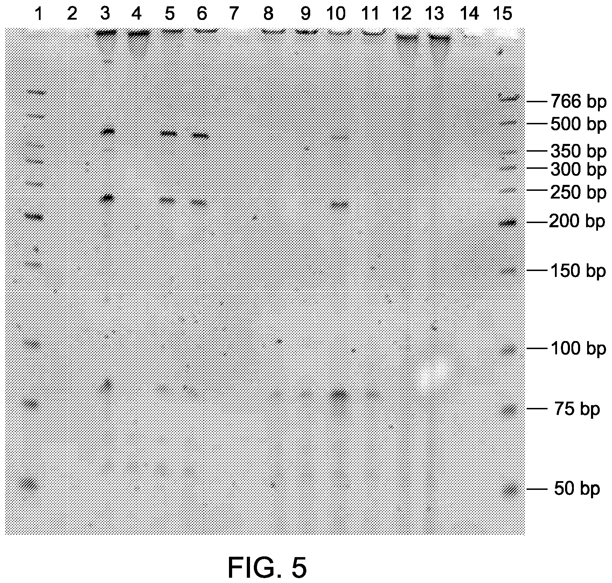

After obtaining the solid medium portion with the fixed tissue sample, the genetic material is directly electroeluted from the solid medium to a subsequent medium (block 54) as described above to obtain high molecular weight DNA of at least 10 kilobases. The extracted genetic material may then be analyzed (block 56) in a variety of ways (e.g., PCR) as demonstrated in FIG. 5.

FIG. 5 illustrates the quality of the DNA extracted from formalin-fixed tissue samples on solid media following crosslink reversal and DNA repair as described above. FIG. 5 depicts a SYBR^{®} Gold stained electrophoresis gel, using a vertical polyacrylamide gel electrophoresis (PAGE) system, of multiplex PCR amplification of DNA that has been extracted from a human prostate sample fixed in formalin and applied to Whatman^{®} FTA^{®} paper as described above. Electrophoresis was in a 6% TBE (Tris-Borate-EDTA)-UREA gel and 1X TBE for 40 minutes at 160V. The fixed human prostate sample was processed as described above (e.g., rehydrated or lysed) and applied to the FTA^{®} paper. The samples on the FTA^{®} paper were then subjected to crosslink reversal and/or DNA repair (with or without endonuclease IV (Endo IV)). After crosslink reversal and/or DNA repair, the solid media is added directly to a PCR reaction containing 3 sets of PCR primers. The sequences of the primers are as follows: 5' CTCACCCTGAAGTTCTCAGG 3' (primer 1), 5' CCTCAAGGGCACCTTTGCCA 3' (primer 2), 5' GTCTACCCTTGGACCCAG 3' (primer 3), and 5' GATGAAGTTGGTGGTGAGG 3' (primer 4). The PCR primers are designed to produce products of 78 base pairs (primers 1 and 2), 218 base pairs (primers 1 and 3), and 380 base pairs (primers 1 and 4) if high molecular weight human DNA is present. Amplification conditions were as follows: step 1: 95°C for 7 minutes; step 2 (for 15 cycles): 95°C for 1 minute, 69°C for 1 minute, and 72°C for 1 minute; step 3 (for 15 cycles): 95°C for 1 minute, 63°C for 1 minute, and 72°C for 1 minute; step 4: 72°C for 10 minutes; and then step 5: 4°C. The samples shown in FIG. 5 are as follows:

Lane 1: low molecular weight DNA ladder (New England Biolabs); Lane 3: purified human DNA (Male, Applied Biosystems); Lane 4: no DNA control; Lane 5: purified human DNA treated with repair reaction (without Endo IV); Lane 6: purified human DNA treated with repair reaction (with Endo IV); Lane 8: formalin-fixed human prostate sample on FTA^{®} paper with crosslink reversal and repair reactions (without Endo IV); Lane 9: formalin-fixed human prostate sample on FTA^{®} paper with repair reaction only (with Endo IV); Lane 10: formalin-fixed human prostate sample on FTA^{®} paper with crosslink reversal and repair reactions (with Endo IV); Lane 11: formalin-fixed human prostate sample on FTA^{®} paper with repair reaction only (with Endo IV); Lane 12: formalin-fixed human prostate sample on FTA^{®} paper with crosslink reversal reaction only; Lane 13: formalin-fixed human prostate sample on FTA^{®} paper without crosslink reversal and repair reactions; and Lane 15: low molecular weight DNA ladder. After PAGE gel electrophoresis, the DNA was stained with SYBR^{®} Gold and the gel was imaged on a Typhoon™ Imager fluorescent scanner.

The results in FIG. 5 demonstrate the appearance of the higher molecular weight PCR products (218 base pairs and 380 base pairs) when the sample on the FTA^{®} paper has been treated to reverse cross-links and to repair DNA (Lane 10) as described above. All other combinations of treatment fail to produce these higher molecular weight PCR products. While the DNA in this experiment has not been eluted from the FTA^{®} paper prior to PCR, it can be appreciated that if the repaired sample still located on the FTA^{®} paper can be used for PCR amplification of higher molecular weight targets, this result in combination with the ability to elute DNA from FTA^{®} paper would allow for a greater range of DNA interrogation experiments to be performed on such samples.

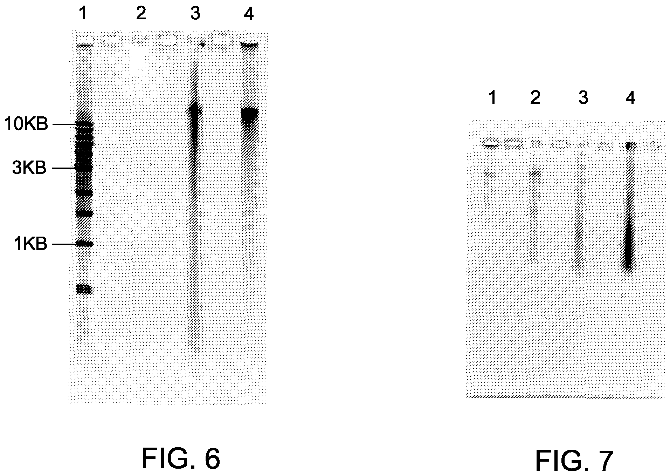

FIGS. 6 and 7 illustrate the benefit of rehydrating fixed samples prior to electroelution from the FTA paper. FIGS. 6 and 7 depict a SYBR^{®} Gold stained electrophoresis gel of DNA electroeluted from solid media using native gel electrophoresis. Electrophoresis was in a 0.8% agarose TBE gel for 80 minutes at 110 volts. The samples shown in FIG. 6 are as follows: Lane 1: 1 kb DNA ladder (New England Biolabs); Lane 2: 0.5 mm slice of formalin-fixed human prostate sample applied directly to FTA^{®} paper, dried, and rinsed in water and electroeluted; Lane 3: 20 µL of Jurkat cells (suspended at 2 x 10⁶ cells/mL) were applied to FTA^{®} paper, dried, and electroeluted; and Lane 4: purified human DNA (Male, Applied Biosystems). The electroeluted samples shown in FIG. 7 are as follows: Lane 1: purified human DNA (Male, Applied Biosystems); Lane 2: 20 µL of Jurkat cells (suspended at 2 x 10⁶ cells/mL) were applied to FTA^{®} paper, and dried; Lane 3: an approximately 0.5 mm slice of formalin-fixed human prostate sample was rehydrated as described above and applied to FTA^{®} paper, dried, and rinsed in water; and Lane 4: approximately 0.5 mm of formalin-fixed human prostate sample was scraped off the paraffin block as a series of flakes, then rehydrated as described above, applied to FTA^{®} paper, dried, and rinsed in water. After TBE-gel electrophoresis, the DNA was stained with SYBR^{®} Gold and the gel was imaged on a Typhoon™ Imager fluorescent scanner.

The results in FIGS. 6 and 7, in particular a comparison between lane 2 of FIG. 6 and lanes 3 and 4 of FIG. 7, clearly indicate that the rehydration process aids in the retrieval of DNA from fixed samples. The inclusion of steps to rehydrate tissue samples that have been dehydrated in preparation for fixation and for paraffin embedding increases the yield of genetic material from the fixed sample upon electroelution.

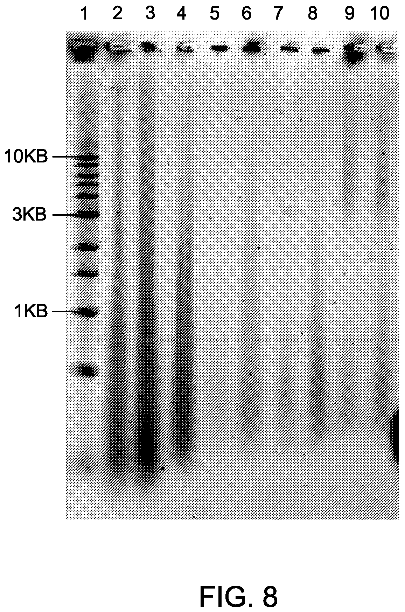

FIG. 8 illustrates the effect of DNA repair on the molecular weight of DNA obtained from fixed samples that have been electroeluted from FTA paper. FIG. 8 depicts a SYBR^{®} Gold stained electrophoresis gel of DNA electroeluted from solid media using native gel electrophoresis. Electrophoresis was in a 0.8% agarose TBE gel for 80 minutes at 110 volts. Samples from formalin-fixed human lung (ILS 19279-A04) were applied to FTA^{®} paper as described above. Cross-links were repaired in 100 mM bicine pH 9.5 and/or treated with a DNA repair reaction in certain samples as described above. The samples were stopped at different stages of the reactions. The samples shown in FIG. 8 are as follows:
Lane 1: 1 kb DNA ladder (New England Biolabs); Lane 2: post rehydration (i.e., ethanol and T.E. washes); Lane 3: post FTA^{®} purification reagent washes; Lane 4: post Proteinase K digestion; Lane 5: post crosslink reversal reaction conducted at 65°C; Lane 6: post crosslink reversal reaction conducted at room temperature; Lane 7: post DNA precipitation (plus crosslink reversal reaction conducted at 65°C); Lane 8: post DNA precipitation (plus crosslink reversal reaction conducted at room temperature); Lane 9: post DNA repair reaction (plus crosslink reversal reaction conducted at 65°C); and Lane 10: post DNA repair reaction (plus crosslink reversal reaction conducted at room temperature).

The results in FIG. 8 demonstrate the appearance of high molecular weight material in lanes 9 and 10 (indicated by the arrow) only after the DNA repair reaction has been used to repair the DNA that is still attached to the FTA^{®} paper. The DNA is repaired while it is still attached to the FTA^{®} paper, before the DNA has been subjected to the forces involved in elution of the DNA from the FTA^{®} paper and the forces that are applied to DNA in solution. This prevents strands of DNA which contain nicks that are located relatively near each other, but on opposite sides, from separating. By repairing these highly nicked and gapped strands, they are prevented from becoming double stranded breaks, and the resulting product is higher molecular weight DNA. The increase in molecular weight provides evidence that the DNA repair reaction was able to repair nicks and gaps in the DNA.

Technical effects of the disclosed embodiments include extracting genetic material (e.g., high molecular weight DNA of at least 10 kilobases) directly from the solid medium via electroelution. The extraction of genetic material occurs in the absence of detergent, thus eliminating additional steps necessary for the removal of detergent prior to any subsequent analysis of the genetic material. Also, the extraction of genetic material avoids the use of high temperatures (e.g., 95°C), thus allowing the extraction of minimally fragmented DNA. The extraction of genetic material, via electroelution directly from the solid medium, applies to both fixed and non-fixed samples. Further, the genetic material may be processed while still on the solid medium. For example, genetic material may be repaired and/or cross-links reversed in fixed cells between the genetic material and protein. Thus, the solid medium provides a single platform for the extraction of genetic material and related processes (e.g., DNA repair) minimizing the work for the user.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method for extracting genetic material from a biological sample stored on a solid medium, comprising:
obtaining the solid medium, wherein the biological sample is applied on the solid medium, and the solid medium comprises chemicals that lyse the biological sample and preserve the genetic material wherein said chemicals comprise a weak base, chelating agent, anionic surfactant or detergent, and/or uric acid or urate salt; and
electroeluting the genetic material directly from the solid medium to a subsequent medium.

2. The method of claim 1, wherein the solid medium comprises a cellulose-based material.

3. The method of claim 1, comprising rinsing the solid medium prior to electroelution.

4. The method of claim 3, wherein rinsing the solid medium comprises soaking the solid medium in buffer or flowing buffer through or across the solid medium.

5. The method of claim 3, wherein rinsing the solid medium comprises soaking the solid medium in water or flowing water through or across the solid medium.

6. The method of claim 3, further comprising repairing the genetic material prior to electroelution.

7. The method of claim 6, wherein repairing the genetic material comprises applying a solution containing at least DNA polymerase and DNA ligase.

8. The method of claim 7, wherein the DNA polymerase comprises DNA polymerase activity, 3' to 5' exonuclease activity for proofreading, and 5' to 3' exonuclease activity for nick translation.

9. The method of claim 6, wherein repairing the genetic material comprises applying a solution containing at least an endonuclease that nicks DNA adjacent to abasic sites.

10. The method of claim 1, wherein the biological sample comprises fixed cells.

11. The method of claim 10, comprising processing the fixed cells prior to electroelution from the solid medium, wherein processing comprises rehydrating the fixed cells, treating the fixed cells with protease, or reversing cross-linking.

12. The method of claim 1, wherein the subsequent medium comprises an electrophoresis gel, a solution, or a capture surface.

13. The method of claim 1 or 2 wherein the biological sample is a fixed tissue sample stored on said solid medium.

14. The method of claim 13, wherein the solid medium comprises FTA paper.

15. The method of claim 13, comprising treating the fixed tissue sample with protease prior to or after applying the fixed tissue sample to the solid medium.

16. The method of claim 13, comprising rehydrating the fixed tissue sample prior to applying the fixed tissue sample to the solid medium.

17. The method of claim 16, comprising reversing cross-linking between the genetic material and protein prior to or subsequent to applying the fixed tissue sample to the solid medium and optionally wherein said reversing cross-linking comprises applying crosslink repair buffer comprising a nucleophile with a pH other than 7.0.

## Patentansprüche

1. Verfahren zum Extrahieren von genetischem Material aus einer auf einem festen Medium gespeicherten biologischen Probe, umfassend:
Herstellen des festen Mediums, wobei die biologische Probe auf das feste Medium aufgebracht wird und das feste Medium Chemikalien umfasst, die die biologische Probe lysieren und das genetische Material bewahren, wobei die Chemikalien eine schwache Base, einen Chelatbildner, ein anionisches Tensid oder ein Detergens und/oder Harnsäure oder Salz der Harnsäure umfassen; und
Elektroeluieren des genetischen Materials direkt von dem festen Medium zu einem nachfolgenden Medium.

2. Verfahren nach Anspruch 1, wobei feste Medium ein Material auf Cellulosebasis umfasst.

3. Verfahren nach Anspruch 1, umfassend ein Spülen des festen Mediums vor dem Elektroeluieren.

4. Verfahren nach Anspruch 3, wobei das Spülen des festen Mediums ein Einweichen des festen Mediums in einem Puffer oder Strömen eines Puffers durch oder über das feste Medium umfasst.

5. Verfahren nach Anspruch 3, wobei das Spülen des festen Mediums ein Einweichen des festen Mediums in Wasser oder Strömen von Wasser durch oder über das feste Medium umfasst.

6. Verfahren nach Anspruch 3, weiter umfassend Reparieren des genetischen Materials vor dem Elektroeluieren.

7. Verfahren nach Anspruch 6, wobei das Reparieren des genetischen Materials ein Aufbringen einer Lösung umfasst, die zumindest DNA-Polymerase und DNA-Ligase aufweist.

8. Verfahren nach Anspruch 7, wobei die DNA-Polymerase eine DNA-Polymerase-Aktivität, eine 3' bis 5' Exonukleaseaktivität zum Korrekturlesen und eine 5' bis 3' Exonukleaseaktivität zur Nick-Translation umfasst.

9. Verfahren nach Anspruch 6, wobei das Reparieren des genetischen Materials ein Aufbringen einer Lösung umfasst, die zumindest eine Endonuklease umfasst, die zu abasischen Stellen benachbarte DNA einschneidet.

10. Verfahren nach Anspruch 1, wobei die biologische Probe fixierte Zellen umfasst.

11. Verfahren nach Anspruch 10, umfassend ein Verarbeiten der fixierten Zellen vor dem Elektroeluieren von dem festen Material, wobei das Verarbeiten ein Rehydratisieren der fixierten Zellen, ein Behandeln der fixierten Zellen mit Protease oder ein Umkehren von Vernetzung umfasst.

12. Verfahren nach Anspruch 1, wobei das nachfolgende Medium ein Elektrophoresegel, eine Lösung oder eine Einfangoberfläche umfasst.

13. Verfahren nach Anspruch 1 oder 2, wobei die biologische Probe eine auf dem festen Medium gespeicherte fixierte Gewebeprobe ist.

14. Verfahren nach Anspruch 13, wobei das feste Medium FTA-Papier umfasst.

15. Verfahren nach Anspruch 13, umfassend ein Behandeln der fixierten Gewebeprobe mit Protease vor oder nach dem Aufbringen der fixierten Gewebeprobe auf das feste Medium.

16. Verfahren nach Anspruch 13, umfassend ein Rehydratisieren der fixierten Gewebeprobe vor dem Aufbringen der fixierten Gewebeprobe auf das feste Medium.

17. Verfahren nach Anspruch 16, umfassend ein Umkehren von Vernetzung zwischen dem genetischen Material und Protein vor oder nach dem Aufbringen der fixierten Gewebeprobe auf das feste Medium und wahlweise, wobei das Umkehren von Vernetzung ein Aufbringen eines Vernetzungsreparaturpuffers umfasst, der ein Nukleophil mit einem PH-Wert umfasst, das von 7,0 verschieden ist.

## Revendications

1. Procédé d'extraction d'une matière génétique d'un échantillon biologique stocké sur un support solide, comprenant :
l'obtention du support solide, dans lequel l'échantillon biologique est appliqué sur le support solide, et le support solide comprend des produits chimiques qui lysent l'échantillon biologique et préservent la matière génétique, dans lequel lesdits produits chimiques comprennent une base faible, un agent chélatant, un tensioactif ou un détergent anionique, et/ou de l'acide urique ou un sel d'urate ; et
l'électroélution de la matière génétique directement du support solide à un support ultérieur.

2. Procédé selon la revendication 1, dans lequel le support solide comprend une matière à base de cellulose.

3. Procédé selon la revendication 1, comprenant le rinçage du support solide avant l'électroélution.

4. Procédé selon la revendication 3, dans lequel le rinçage du support solide comprend l'immersion du support solide dans un tampon ou l'écoulement d'un tampon à travers ou en travers du support solide.

5. Procédé selon la revendication 3, dans lequel le rinçage du support solide comprend l'immersion du support solide dans de l'eau ou l'écoulement d'eau à travers ou en travers du support solide.

6. Procédé selon la revendication 3, comprenant en outre la réparation de la matière génétique avant l'électroélution.

7. Procédé selon la revendication 6, dans lequel la réparation de la matière génétique comprend l'application d'une solution contenant au moins une polymérase d'ADN et une ligase d'ADN.

8. Procédé selon la revendication 7, dans lequel la polymérase d'ADN comprend une activité de polymérase d'ADN, une activité d'exonucléase 3' à 5' pour la correction, et une activité d'exonucléase 5' à 3' pour le déplacement de la coupure simple brin.

9. Procédé selon la revendication 6, dans lequel la réparation de la matière génétique comprend l'application d'une solution contenant au moins une endonucléase qui entaille l'ADN adjacent aux sites abasiques.

10. Procédé selon la revendication 1, dans lequel l'échantillon biologique comprend des cellules fixées.

11. Procédé selon la revendication 10, comprenant le traitement des cellules fixées avant électroélution du support solide, dans lequel le traitement comprend la réhydratation des cellules fixées, le traitement des cellules fixées avec une protéase, et l'inversion de la réticulation.

12. Procédé selon la revendication 1, dans lequel le support ultérieur comprend un gel d'électrophorèse, une solution ou une surface de capture.

13. Procédé selon la revendication 1 ou 2, dans lequel l'échantillon biologique est un échantillon de tissu fixé stocké sur ledit support solide.

14. Procédé selon la revendication 13, dans lequel le support solide comprend du papier FTA.

15. Procédé selon la revendication 13, comprenant le traitement de l'échantillon de tissu fixé avec une protéase avant ou après l'application de l'échantillon de tissu fixé au support solide.

16. Procédé selon la revendication 13, comprenant la réhydratation de l'échantillon de tissu fixé avant d'appliquer l'échantillon de tissu fixé au support solide.

17. Procédé selon la revendication 16, comprenant l'inversion de la réticulation entre la matière génétique et la protéine avant ou après l'application de l'échantillon de tissu fixé au support solide et éventuellement dans lequel ladite inversion de réticulation comprend l'application d'un tampon de réparation de réticulation comprenant un nucléophile avec un pH autre que 7,0.


### SEQUENCE LISTING

<110> Finehout, Erin J. Nelson, John R. Spooner, Patrick M. GENERAL ELECTRIC COMPANY
<120> METHOD FOR ELECTROELUTING GENETIC MATERIAL FROM DRIED SAMPLES
<130> PG11025
<140> PCT/US2011/to be assigned
   <141> 2011-12-14
<150> 12/972,236
   <151> 2010-12-17
<160> 4
<170> PatentIn version 3.5
<210> 1
   <211> 20
   <212> DNA
   <213> Homo sapiens
<400> 1
   ctcaccctga agttctcagg 20
<210> 2
   <211> 20
   <212> DNA
   <213> Homo sapiens
<400> 2
   cctcaagggc acctttgcca 20
<210> 3
   <211> 18
   <212> DNA
   <213> Homo sapiens
<400> 3
   gtctaccctt ggacccag 18
<210> 4
   <211> 19
   <212> DNA
   <213> Homo sapiens
<400> 4
   gatgaagttg gtggtgagg 19